# EUROPÄISCHE PATENTSCHRIFT

(11) **EP 0 608 759 B1**
(45) Veröffentlichungstag und Bekanntmachung des Hinweises auf die Patenterteilung: **22.08.2001**
(21) Anmeldenummer: 94100709.8
(22) Anmeldetag: 19.01.1994
(51) Int. Cl.: C07D 241/08, C07D 295/185, C07D 295/215, C07D 295/192, A61K 31/495

(54) **Piperazinderivate**
Piperazine derivatives
Dérivés de pipérazines

(30) Priorität: 29.01.1993 DE 4302485
(43) Veröffentlichungstag der Anmeldung: 03.08.1994
(73) Patentinhaber: MERCK PATENT GmbH, 64293 Darmstadt (DE)
(72) Erfinder: Gante, Joachim, Prof.Dr., D-64291 Darmstadt (DE); Raddatz, Peter, Dr., D-64342 Seeheim (DE); Juraszyk, Horst, Dr., D-64342 Seeheim (DE); Bernotat-Danielowski, Sabine, Dr., D-61231 Bad-Nauheim (DE); Melzer, Guido, Dr., D-65719 Hofheim (DE)

(56) Entgegenhaltungen:
- EP-A- 0 284 942
- EP-A- 0 381 033
- EP-A- 0 446 706
- EP-A- 0 462 960
- EP-A- 0 529 858

## Beschreibung

Die Erfindung betrifft neue Verbindungen der Formel I

Y- (CₘH₂ₘ-CHR¹)ₙ-CO-(NH-CHR²-CO)ᵣ-Z I

worin
- Y: 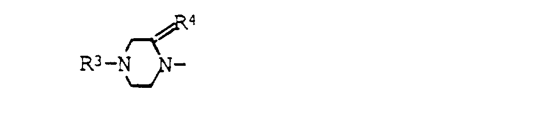
- Z: 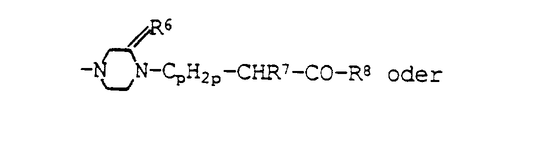 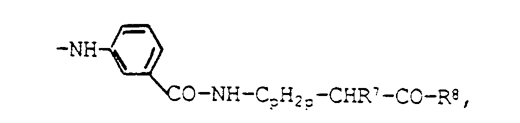
- R¹, R² und R⁷: jeweils -CₜH₂ₜ-R⁹, Benzyl, Hydroxybenzyl, Imidazolyl-methyl oder Indolyl-methyl,
- R³: H oder H₂N-C(=NH)-,
- R⁴ und R⁶: jeweils (H,H) oder =O,
- R⁸: OH, OA oder NHOH,
- R⁹: H, OH, NH₂, SH, SA, COOH, CONH₂ oder NH-C (=NH)-NH₂,
- A: jeweils Alkyl mit 1-4 C-Atomen,
- m und t: jeweils 0, 1, 2, 3 oder 4,
- n und r: jeweils 0 oder 1 und
- p: 0, 1 oder 2 bedeuten,
worin ferner die Piperazinringe durch 1 bis 4 Gruppen A substituiert sein können, sowie deren Salze.

Ähnliche Verbindungen sind aus der EP-A1-0 381 033 bekannt.

Der Erfindung lag die Aufgabe zugrunde, neue Verbindungen mit wertvollen Eigenschaften aufzufinden, insbesondere solche, die zur Herstellung von Arzneimitteln verwendet werden können.

Diese Aufgabe wurde durch die Erfindung gelöst. Es wurde gefunden, daß die Verbindungen der Formel I sowie ihre Solvate und Salze bei guter Verträglichkeit wertvolle pharmakologische Eigenschaften besitzen. Insbesondere hemmen sie die Bindung von Fibrinogen, Fibronectin und des von Willebrand-Faktors an den Fibrinogenrezeptor der Blutplättchen (Glykoprotein IIb/IIIa) als auch die Bindung derselben und weiterer adhäsiver Proteine, wie Vitronectin, Kollagen und Laminin, an die entsprechenden Rezeptoren auf der Oberfläche verschiedener Zelltypen. Die Verbindungen beeinflussen somit Zell-Zell- und Zell-Matrix-Wechselwirkungen. Sie verhindern insbesondere die Entstehung von Blutplättchenthromben und können daher zur Behandlung von Thrombosen, Apoplexie, Herzinfarkt, Entzündungen, Arteriosklerose verwendet werden. Ferner haben die Verbindungen einen Effekt auf Tumorzellen, indem sie deren Metastasierung hemmen. Somit können sie auch als Anti-Tumor-Mittel eingesetzt werden.

Die Eigenschaften der Verbindungen können nach Methoden nachgewiesen werden, die in der EP-A1-0 462 960 beschrieben sind. Die Hemmung der Fibrinogenbindung an den Fibrinogenrezeptor kann nach der Methode nachgewiesen werden, die in der EP-A1-0 381 033 angegeben ist. Die thrombozytenaggregationshemmende Wirkung läßt sich in vitro nach der Methode von Born (Nature 4832, 927-929, 1962) nachweisen.

Gegenstand der Erfindung ist ferner ein Verfahren zur Herstellung einer Verbindung der angegebenen Formel I sowie von deren Salzen, dadurch gekennzeichnet, daß man
(a) eine Verbindung der Formel I aus einem ihrer funktionellen Derivate durch Behandeln mit einem solvolysierenden oder hydrogenolysierenden Mittel in Freiheit setzt, oder daß man
(b) eine Carbonsäure der Formel II

   Y- (CₘH₂ₘ-CHR¹)ₙ-CO-G¹-OH II

   worin
   - G¹: (a) fehlt,
   (b) -NH-CHR²-CO- bedeutet,
   (c) 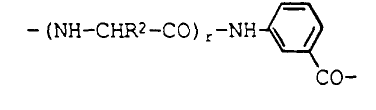 bedeutet,
   Y, R¹, R², m, n und r die oben angegebenen Bedeutungen haben
oder eines ihrer reaktionsfähigen Derivate
mit einer Aminoverbindung der Formel III

H-G² III

worin
- G²: (a) -(NH-CHR²-CO)ᵣ-Z bedeutet,
(b) Z bedeutet,
(c) -NH-CₚH₂ₚ-CHR⁷-CO-R⁸ bedeutet, und
Z, R², R⁷, R⁸, r und p die oben angegebenen Bedeutungen haben,
umsetzt
oder daß man
(c) zur Herstellung einer Verbindung der Formel I, worin R³ H2N-C(=NH)- bedeutet, an ein Nitril, das der Formel I entspricht, aber an Stelle des Restes R³ eine CN-Gruppe enthält, Ammoniak anlagert,
und/oder daß man gegebenenfalls eine Carbonsäure der Formel I (R⁸ = OH) in einen entsprechenden Ester (I, R⁸ = OA) oder in die entsprechende Hydroxamsäure (I, R⁸ = NHOH) überführt und/oder ein H-Atom durch Behandeln mit einem amidinierenden Mittel durch eine Amidinogruppe ersetzt oder einen Ester der Formel I (R⁸ = OA) verseift und/oder eine Verbindung der Formel I durch Behandeln mit einer Säure oder einer Base in eines ihrer Salze überführt.

Einzelne der Verbindungen der Formel I besitzen chirale Zentren und können daher in mehreren enantiomeren Formen auftreten. Alle diese Formen (z.B. D- und L-Formen) und deren Gemische (z.B. die DL-Formen) sind in der Formel I eingeschlossen.

Vor- und nachstehend haben die Reste bzw. Parameter Y, Z, R¹ bis R⁹, A, G¹, G², m, n, p, r und t die bei den Formeln I, II oder III angegebenen Bedeutungen, falls nicht ausdrücklich etwas anderes angegeben ist.

Die Gruppen A bedeuten jeweils Alkylgruppen mit 1-4, vorzugsweise 1 oder 2 C-Atomen, insbesondere Methyl oder Ethyl, ferner Propyl, Isopropyl, Butyl, Isobutyl, sek.-Butyl oder tert.-Butyl. Falls mehrere Gruppen A in der Verbindung der Formel I vorhanden sind, können sie gleich oder voneinander verschieden sein.

Der Parameter m ist vorzugsweise 0 oder 1; t ist vorzugsweise 0, aber auch 1, 2, 3 oder 4; n ist vorzugsweise 1, aber auch 0; r ist vorzugsweise 0, aber auch 1; p ist vorzugsweise 1, aber auch 0 oder 2.

Die Gruppen -NH-CHR²-CO- und -NH-CₚH₂ₚ-CHR⁷-CO- können insbesondere für die Reste von natürlich vorkommenden Aminosäure stehen, vorzugsweise für -NH-CH₂-CO- (Glycin-Rest), aber auch für die Reste von L- oder D-Alanin, L- oder D-Valin, L- oder D-Leucin, L- oder D-Isoleucin, L- oder D-Phenylalanin, L- oder D-Tyrosin, L- oder D-Histidin, L- oder D-Tryptophan, Loder D-Serin, L- oder D-Threonin, L- oder D-Ornithin, L- oder D-Lysin, L- oder D-Cystein, L- oder D-Methionin, L- oder D-Asparaginsäure, L- oder D-Asparagin, L- oder D-Arginin. Die Gruppe -NH-CₚH₂ₚ-CHR⁷-CO- steht bevorzugt auch für -NH-CH₂CH₂-CO- (Rest des β-Alanins) oder für -NH-(CH₂)₃-CO- (Rest der 4-Aminobuttersäure).

R¹, R², R⁷ oder R⁹ sind vorzugsweise jeweils H. R³ ist vorzugsweise H₂N-C(=NH)- (Amidino). R⁴ und R⁶ sind vorzugsweise jeweils (H,H).
R⁸ ist vorzugsweise OH, ferner bevorzugt OA, insbesondere OCH₃ oder OC₂H₅.
Dementsprechend ist die Gruppe -(CₘH₂ₘ-CHR¹)ₙ-CO-(NH-CHR²-CO)ᵣ-vorzugsweise -CO-, -CH₂-CO-, -CO-NH-CH₂-CO- oder -CH₂CH₂-CO-.

Die Piperazinringe können zusätzlich durch 1-4 Gruppen A, vorzugsweise durch 1-4 Methylgruppen substituiert sein. Bevorzugte substituierte Piperazinringe sind 2-Methyl-1,4-piperazin-diyl, 2,5-Dimethyl-1,4-piperazin-diyl, 2,6-Dimethyl-1,4-piperazin-diyl und 2,3,5,6-Tetramethyl-1,4-piperazin-diyl.

Unter den Verbindungen der Formel I sind diejenigen bevorzugt, in denen mindestens eine(r) der angegebenen Reste, Gruppen und/oder Parameter eine der angegebenen bevorzugten Bedeutungen hat. Einige Gruppen von bevorzugten Verbindungen sind diejenigen der Formeln Ia bis Id, die der Formel I entsprechen und worin die nicht näher angegebenen Reste und/oder Parameter die bei Formel I angegebenen Bedeutungen haben, worin jedoch
in Ia
   - Y: 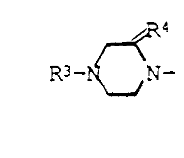 bedeutet;
in Ib
   - Y: 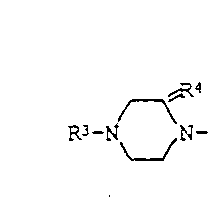 und
   - Z: 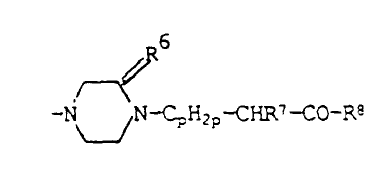 bedeuten;
in Ic
   - Y: 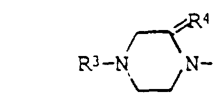 und
   - Z: 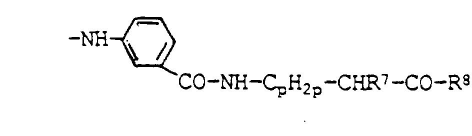 bedeuten;
in Id
   - Y: 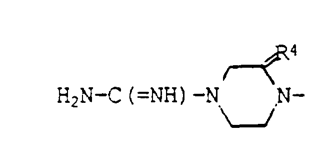 bedeutet.

Weiterhin sind bevorzugt Verbindungen der Formeln Ie sowie Iae bis Ide, die den Formeln I sowie Ia bis Id entsprechen, worin jedoch zusätzlcih die Gruppe -(CₘH₂ₘ-CHR¹)ₙ-CO-(NH-CHR²-CO)ᵣ-
-CO-, -CH₂-CO-, -CO-NH-CH₂-CO- oder -CH₂CH₂CO- bedeutet.

Ferner sind bevorzugt Verbindungend der Formeln If, Iaf bis Idf sowie Iaef bis Idef, die den Formeln I, Ia bis Id sowie Iae bis Ide entsprechen, worin jedoch zusätzlich die Gruppe -CₚH₂ₚ-CHR⁷-CO-R⁸
-CH₂COOH, -CH₂CH₂COOH, - (CH₂)₃-COOH, -CH (COOH) -CH₂COOH, -CH₂COOA, -CH₂CH₂COOA, -(CH₂)₃-COOA oder -CH (COOA) -CH₂COOA bedeutet.

Die Verbindungen der Formel I und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart; ferner EP-A1-0381033, EP-A1-0462960) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können, falls erwünscht, auch in situ gebildet werden, so daß man sie aus dem Reaktionsgemisch nicht isoliert, sondern sofort weiter zu den Verbindungen der Formel I umsetzt.

Die Verbindungen der Formel I können erhalten werden, indem man sie aus ihren funktionellen Derivaten durch Solvolyse, insbesondere Hydrolyse, oder durch Hydrogenolyse in Freiheit setzt.

Bevorzugte Ausgangsstoffe für die Solvolyse bzw. Hydrogenolyse sind solche, die sonst der Formel I entsprechen, aber an Stelle einer oder mehrerer freier Amino- und/oder Hydroxygruppen entsprechende geschützte Amino- und/oder Hydroxygruppen enthalten, vorzugsweise solche, die an Stelle eines H-Atoms, das mit einem N-Atom verbunden ist, eine Aminoschutzgruppe tragen, insbesondere solche, die an Stelle einer H₂N-Gruppe eine R'-NH-Gruppe tragen, worin R' eine Aminoschutzgruppe bedeutet, und/oder solche, die an Stelle des H-Atoms einer Hydroxygruppe eine Hydroxyschutzgruppe tragen, z.B. solche, die der Formel I entsprechen, jedoch an Stelle einer Gruppe -CₜH₂ₜ-R⁹ eine Gruppe -CₜH₂ₜ-OR" und/oder an Stelle einer Gruppe R⁸ eine Gruppe -OR" tragen, worin R" eine Hydroxyschutzgruppe bedeutet.

Es können auch mehrere - gleiche oder verschiedene - geschützte Amino- und/oder Hydroxygruppen im Molekül des Ausgangsstoffes vorhanden sein. Falls die vorhandenen Schutzgruppen voneinander verschieden sind, können sie in vielen Fällen selektiv abgespalten werden.

Der Ausdruck "Aminoschutzgruppe" ist allgemein bekannt und bezieht sich auf Gruppen, die geeignet sind, eine Aminogruppe vor chemischen Umsetzungen zu schützen (zu blockieren), die aber leicht entfernbar sind, nachdem die gewünschte chemische Reaktion an einer anderen Stelle des Moleküls durchgeführt worden ist. Typisch für solche Gruppen sind insbesondere unsubstituierte oder substituierte Acyl-, Aryl- (z.B. 2,4-Dinitrophenyl (DNP)), Aralkoxymethyl- (z.B. Benzyloxymethyl (BOM)) oder Aralkyl-gruppen (z.B. Benzyl, 4-Nitrobenzyl, Triphenylmethyl). Da die Aminoschutzgruppen nach der gewünschten Reaktion (oder Reaktionsfolge) entfernt werden, ist ihre Art und Größe im übrigen nicht kritisch; bevorzugt werden jedoch solche mit 1-20, insbesondere 1-8 C-Atomen. Der Ausdruck "Acylgruppe" ist im Zusammenhang mit dem vorliegenden Verfahren im weitesten Sinne aufzufassen. Er umschließt von aliphatischen, araliphatischen, aromatischen oder heterocyclischen Carbonsäuren oder Sulfonsäuren abgeleitete Acylgruppen sowie insbesondere Alkoxycarbonyl-, Aryloxycarbonyl- und vor allem Aralkoxycarbonylgruppen. Beispiele für derartige Acylgruppen sind Alkanoyl wie Acetyl, Propionyl, Butyryl; Aralkanoyl wie Phenylacetyl; Aroyl wie Benzoyl oder Toluyl; Aryloxyalkanoyl wie Phenoxyacetyl; Alkoxycarbonyl wie Methoxycarbonyl, Ethoxycarbonyl, 2,2,2-Trichlorethoxycarbonyl, Isopropoxycarbonyl, tert.-Butoxycarbonyl (BOC), 2-Jodethoxycarbonyl; Aralkyloxycarbonyl wie Benzyloxycarbonyl (CBZ), 4-Methoxybenzyloxycarbonyl, 9-Fluorenylmethoxycarbonyl (FMOC). Bevorzugte Aminoschutzgruppen sind BOC, DNP und BOM, ferner CBZ, Benzyl und Acetyl.

Der Ausdruck "Hydroxyschutzgruppe" ist ebenfalls allgemein bekannt und bezieht sich auf Gruppen, die geeignet sind, eine Hydroxygruppe vor chemischen Umsetzungen zu schützen, die aber leicht entfernbar sind, nachdem die gewünschte chemische Reaktion an einer anderen Stelle des Moleküls durchgeführt worden ist. Typisch für solche Gruppen sind die oben genannten unsubstituierten oder substituierten Aryl-, Aralkyl- oder Acylgruppen, ferner auch Alkylgruppen. Die Natur und Größe der Hydroxyschutzgruppen ist nicht kritisch, da sie nach der gewünschten chemischen Reaktion oder Reaktionsfolge wieder entfernt werden; bevorzugt sind Gruppen mit 1-20, insbesondere 1-10 C-Atomen. Beispiele für Hydroxyschutzgruppen sind u.a. tert.-Butyl, Benzyl, p-Nitrobenzoyl, p-Toluolsulfonyl und Acetyl, wobei Benzyl und Acetyl besonders bevorzugt sind.

Die als Ausgangsstoffe zu verwendenden funktionellen Derivate der Verbindungen der Formel I können nach üblichen Methoden der Aminosäure- und Peptidsynthese hergestellt werden, wie sie z.B. in den genannten Standardwerken und Patentanmeldungen beschrieben sind, z.B. auch nach der Festphasenmethode nach Merrifield.

Das In-Freiheit-Setzen der Verbindungen der Formel I aus ihren funktionellen Derivaten gelingt - je nach der benutzten Schutzgruppe - z.B. mit starken Säuren, zweckmäßig mit Trifluoressigsäure oder Perchlorsäure, aber auch mit anderen starken anorganischen Säuren wie Salzsäure oder Schwefelsäure, starken organischen Carbonsäuren wie Trichloressigsäure oder Sulfonsäuren wie Benzol- oder p-Toluolsulfonsäure. Die Anwesenheit eines zusätzlichen inerten Lösungsmittels ist möglich, aber nicht immer erforderlich.

Als inerte Lösungsmittel eignen sich vorzugsweise organische, beispielsweise Carbonsäuren wie Essigsäure, Ether wie Tetrahydrofuran oder Dioxan, Amide wie Dimethylformamid (DMF), halogenierte Kohlenwasserstoffe wie Dichlormethan, ferner auch Alkohole wie Methanol, Ethanol oder Isopropanol sowie Wasser. Ferner kommen Gemische der vorgenannten Lösungsmittel in Frage. Trifluoressigsäure wird vorzugsweise im Überschuß ohne Zusatz eines weiteren Lösungsmittels verwendet, Perchlorsäure in Form eines Gemisches aus Essigsäure und 70%iger Perchlorsäure im Verhältnis 9:1. Die Reaktionstemperaturen für die Spaltung liegen zweckmäßig zwischen etwa 0 und etwa 50°; vorzugsweise arbeitet man zwischen 15 und 30° (Raumtemperatur).

Die BOC-Gruppe kann z.B. bevorzugt mit 40%iger Trifluoressigsäure in Dichlormethan oder mit etwa 3 bis 5 n HCl in Dioxan bei 15-60° abgespalten weden, die FMOC-Gruppe mit einer etwa 5-20%igen Lösung von Dimethylamin, Diethylamin oder Piperidin in DMF bei 15-50°. Eine Abspaltung der DNP-Gruppe gelingt z.B. auch mit einer etwa 3-10%igen Lösung von 2-Mercaptoethanol in DMF/Wasser bei 15-30°.

Hydrogenolytisch entfernbare Schutzgruppen (z.B. BOM, CBZ oder Benzyl) können z.B. durch Behandeln mit Wasserstoff in Gegenwart eines Katalysators (z.B. eines Edelmetallkatalysators wie Palladium, zweckmäßig auf einem Träger wie Kohle) abgespalten werden. Als Lösungsmittel eignen sich dabei die oben angegebenen, insbesondere z.B. Alkohole wie Methanol oder Ethanol oder Amide wie DMF. Die Hydrogenolyse wird in der Regel bei Temperaturen zwischen etwa 0 und 100° und Drucken zwischen etwa 1 und 200 bar, bevorzugt bei 20-30° und 1-10 bar durchgeführt. Eine Hydrogenolyse der CBZ-Gruppe gelingt z.B. gut an 5-10%igem Pd-C in Methanol bei 20-30°.

Verbindungen der Formel I können auch durch direkte Kondensation aus einer Carbonsäure- (Formel II) und einer Aminokomponente (Formel III) erhalten werden. Als Carbonsäurekomponenten eignen sich z.B. solche der Teilformeln
(a) Y-(CₘH₂ₘ-CHR¹)ₙ-COOH,
(b) Y-(CₘH₂ₘ-CHR¹)ₙ-CO-NH-CHR²-COOH oder
(C) Y- (CₘH₂ₘ-CHR¹)ₙ-CO-(NH-CHR²-CO)ᵣ 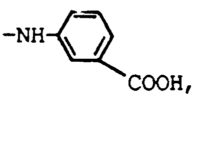
als Aminokomponenten solche der Teilformeln
(a) H-(NH-CHR²-CO)ᵣ-Z,
(b) H-Z oder
(c) H₂N-CₚH₂ₚ-CHR⁷-CO-R⁸.

Dabei arbeitet man zweckmäßig nach üblichen Methoden der Peptid-Synthese, wie sie z.B. in Houben-Weyl, 1.c., Band 15/II, Seiten 1-806 (1974) beschrieben sind.

Die Reaktion gelingt vorzugsweise in Gegenwart eines Dehydratisierungsmittels, z.B. eines Carbodiimids wie Dicyclohexylcarbodiimid (DCCI) oder N-Dimethylaminopropyl-N'-ethyl-carbodiimid (DAPECI), ferner Propanphosphonsäureanhydrid (vgl. Angew.Chem. 92, 129 (1980)), Diphenylphosphorylazid oder 2-Ethoxy-N-ethoxycarbonyl-1,2-dihydrochinolin, in einem inerten Lösungsmittel, z.B. einem halogenierten Kohlenwasserstoff wie Dichlormethan, einem Ether wie THF oder Dioxan, einem Amid wie DMF oder Dimethylacetamid, einem Nitril wie Acetonitril, bei Temperaturen zwischen etwa -10 und 40, vorzugsweise zwischen 0 und 30°.

An Stelle von II bzw. III können auch geeignete reaktionsfähige Derivate dieser Stoffe in die Reaktion eingesetzt werden, z.B. solche, in denen reaktive Gruppen intermediär durch Schutzgruppen blockiert sind. Die Säurederivate II können z.B. in Form ihrer aktivierten Ester verwendet werden, die zweckmäßig in situ gebildet werden, z.B. durch Zusatz von HOBt oder N-Hydroxysuccinimid.

Zur Herstellung eines Amidins der Formel I (R³ = H₂N-C(=NH)-) kann man ferner an ein Nitril, das der Formel I entspricht, aber an Stelle von R³ eine CN-Gruppe enthält, Ammoniak anlagern. Die Anlagerung erfolgt bevorzugt mehrstufig, indem man in an sich bekannter Weise a) das Nitril mit H₂S in ein Thioamid umwandelt, das mit einem Alkylierungsmittel, z.B. CH₃I, in den entsprechenden S-Alkylimidothioester übergeführt wird, welcher seinerseits mit NH₃ zum Amidin reagiert, b) das Nitril mit einem Alkohol, z.B. Ethanol in Gegenwart von HCl in den entsprechenden Imidoester umwandelt und diesen mit Ammoniak behandelt, oder c) das Nitril mit Lithium-bis-(trimethylsilyl)-amid umsetzt und das Produkt anschließend hydrolysiert.

Die Ausgangsstoffe für die angegebenen Verfahrensvarianten, z.B. diejenigen der Formeln II und III sind teilweise bekannt. Sofern sie nicht bekannt sind, können sie nach bekannten Methoden, z.B. den oben angegebenen Methoden der Kondensation und der Abspaltung von Schutzgruppen hergestellt werden.

Gewünschtenfalls kann in einer Verbindung der Formel I eine Carbonsäuregruppe verestert oder in eine Hydroxamsäuregruppe übergeführt oder eine Estergruppe verseift werden.

Zur Veresterung kann man eine Säure der Formel I (R⁸ = OH) mit einem Überschuß eines Alkohols der Formel A-OH behandeln, zweckmäßig in Gegenwart einer starken Säure wie Salzsäure oder Schwefelsäure bei Temperaturen zwischen 0 und 100, vorzugsweise 20 und 50°.

Zur Herstellung von Hydroxamsäuren der Formel I (R⁸ = NHOH) behandelt man zweckmäßig einen entsprechenden Ester der Formel I (R⁸ = OA) mit Hydroxylamin, das aus einem seiner Salze, z.B. dem Hydrochlorid, mit einem Alkalimetallalkoholat, z.B. Natriumethylat, freigesetzt werden kann. Man arbeitet zweckmäßig in Gegenwart eines inerten Lösungsmittels, z.B. eines Alkohols wie Methanol, Ethanol oder Isopropanol bei Temperaturen zwischen etwa 0 und 40, vorzugsweise zwischen 15 und 30°.

Ferner kann eine Verbindung der Formel I, worin R⁸ OA bedeutet, in die entsprechende Verbindung der Formel I umgewandelt werden, worin R⁸ OH bedeutet, zweckmäßig durch selektive Solvolyse nach einer der oben angegebenen Methoden, z.B. mit NaOH oder KOH in Wasser-Dioxan bei Temperaturen zwischen 0 und 40°, vorzugsweise 10 und 30°.

Ferner kann in einer Verbindung der Formel Iein H-Atom durch Behandeln mit einem amidinierenden Mittel durch eine Amidinogruppe ersetzt werden. Als Ausgangsstoffe eignen sich bevorzugt Piperazinderivate, worin R³ = H ist; als amidinierendes Mittel ist 1-Amidino-3,5-dimethylpyrazol bevorzugt, das insbesondere in Form seines Nitrats eingesetzt wird. Man arbeitet zweckmäßig unter Zusatz einer Base wie Triethylamin der Ethyl-diisopropylamin in einem inerten Lösungsmittel oder Lösungsmittelgemisch, z.B. Wasser/Dioxan bei Temperaturen zwischen 0 und 120°, vorzugsweise 60 und 120°.

Eine Base der Formel I kann mit einer Säure in das zugehörige Säureadditionssalz übergeführt werden. Für diese Umsetzung kommen insbesondere Säuren in Frage, die physiologisch unbedenkliche Salze liefern, So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Trifluoressigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Citronensäure, Gluconsäure, Ascorbinsäure, Nicotinsäure, Isonicotin- säure, Methan- oder Ethansulfonsäure, Ethandisulfonsäure, 2-Hydroxyethansulfonsäure, Benzolsulfonsäure, p-Toluolsulfonsäure, Naphthalin-mono- und -disulfonsäuren, Laurylschwefelsäure. Salze mit physiologisch nicht unbedenklichen Säuren, z.B. Pikrate, können zur Isolierung und/oder Aufreinigung der Verbindungen der Formel I verwendet werden.

Die neuen Verbindungen der Formel I und ihre physiologisch unbedenklichen Salze können zur Herstellung pharmazeutischer Präparate verwendet werden, indem man sie zusammen mit mindestens einem Träger- oder Hilfsstoff und, falls erwünscht, zusammen mit einem oder mehreren weiteren Wirkstoff(en) in eine geeignete Dosierungsform bringt. Die so erhaltenen Zubereitungen können als Arzneimittel in der Human- oder Veterinärmedizin eingesetzt werden. Als Trägersubstanzen kommen organische oder anorganische Stoffe in Frage, die sich für die enterale (z.B. orale oder rektale) oder parenterale Applikation oder für eine Applikation in Form eines Inhalations-Sprays eignen und mit den neuen Verbindungen nicht reagieren, beispielsweise Wasser, pflanzliche Öle, Benzylalkohole, Polyethylenglykole, Glycerintriacetat und andere Fettsäureglyceride, Gelatine, Sojalecithin, Kohlehydrate wie Lactose oder Stärke, Magnesiumstearat, Talk, Cellulose. Zur oralen Anwendung dienen insbesondere Tabletten, Dragees, Kapseln, Sirupe, Säfte oder Tropfen; von Interesse sind speziell Lacktabletten und Kapseln mit magensaftresistenten Überzügen bzw. Kapselhüllen. Zur rektalen Anwendung dienen Suppositorien, zur parenteralen Applikation Lösungen, vorzugsweise ölige oder wässerige Lösungen, ferner Suspensionen, Emulsionen oder Implantate.

Für die Applikation als Inhalations-Spray können Sprays verwendet werden, die den Wirkstoff entweder gelöst oder suspendiert in einem Treibgasgemisch enthalten. Zweckmäßig verwendet man den Wirkstoff dabei in mikronisierter Form, wobei ein oder mehrere zusätzliche physiologisch verträgliche Lösungsmittel zugegen sein können, z.B. Ethanol. Inhalationslösungen können mit Hilfe üblicher Inhalatoren verabfolgt werden. Die neuen Verbindungen können auch lyophilisiert und die erhaltenen Lyophilisate z.B. zur Herstellung von Injektionspräparaten verwendet werden. Die angegebenen Zubereitungen können sterilisiert sein und/oder Hilfsstoffe wie Konservierungs-, Stabilisierungs- und/oder Netzmittel, Emulgatoren, Salze zur Beeinflussung des osmotischen Druckes, Puffersubstanzen, Farb- und/oder Aromastoffe enthalten. Sie können, falls erwünscht, auch einen oder mehrere weitere Wirkstoffe enthalten, z.B. ein oder mehrere Vitamine.

Die erfindungsgemäßen Substanzen werden in der Regel in Analogie zu anderen bekannten, im Handel befindlichen Peptiden, insbesondere aber in Analogie zu den in der EP-A-249096 beschriebenen Verbindungen verabreicht, vorzugsweise in Dosierungen zwischen etwa 5 mg und 1 g, insbesondere zwischen 50 und 500 mg pro Dosierungseinheit. Die tägliche Dosierung liegt vorzugsweise zwischen etwa 0,1 und 20 mg/kg, insbesondere 1 und 10 mg/kg Körpergewicht. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

Vor- und nachstehend sind alle Temperaturen in °C angegeben. In den nachfolgenden Beispielen bedeutet "übliche Aufarbeitung": Man gibt, falls erforderlich, Wasser hinzu, stellt je nach Konstitution des Endprodukts auf pH-Werte zwischen 2 und 8 ein, extrahiert mit Ethylacetat oder Dichlormethan, trennt ab, trocknet die organische Phase über Natriumsulfat, dampft ein und reinigt durch Chromatographie an Kieselgel und/oder Kristallisation. FAB = (M⁺ + 1) Peak im Massenspektrum, erhalten nach der "Fast Atom Bombardment"-Methode.

### Beispiel 1

Eine Lösung von 1 g
3-(3-(4-CBZ-amidino-1-piperazinyl-acetamido)-benzamido)propionsäure-benzylester (FAB 601; erhältlich durch Reaktion von 1-Piperazinyl-essigsäure-tert.-butylester mit N-CBZ-S-methyl-isothioharnstoff zu 4-CBZ-amidino-1-piperazinyl-essigsäure-tert.-butylester, Abspaltung der tert.-Butylgruppe mit 4 n HCl im Dioxan und Kondensation der erhaltenen 4-CBZ-amidino-1-piperazinyl-essigsäure mit 3-(3-Amino-benzamido)propionsäure-benzylester) in einem Gemisch von 38 ml Methanol, 6 ml Wasser und 6 ml Essigsäure wird an 0,6 g 5%ig. Pd-C bei 20° und 1 bar bis zum Ende der H₂-Aufnahme hydriert. Man filtert, dampft das Filtrat ein, kristallisiert den Rückstand aus Ethylacetat um und erhält 3- (3- (4-Amidino-1-piperazinyl-acetamido)-benzamido)-propionsäure, F. 270° (Zers.);
   Analog erhält man durch Hydrogenolyse:
aus 3-(3-(4-CBZ-1-piperazinyl-carboxamido-acetamido)-benzamido)-propionsäure-benzylester (FAB 602; erhältlich durch Reaktion von 1-CBZ-piperazin mit 3-(3-(Isocyanatoacetamido)benzamido)-propionsäurebenzylester) :
   3-(3-(1-Piperazinyl-carboxamido-acetamido)-benzamido)-propionsäure, F. 123°;
aus 3-(3-(4-CBZ-amidino-1-piperazinyl-carboxamido-acetamido)-benzamido)-propionsäure-benzylester (FAB 664; erhältlich durch Reaktion von 1-CBZ-amidino-piperazin mit 3-(3-Isocyanato-acetamido-benzamido) -propionsäure-benzylester: 3-(3-(4-Amidino-1-piperazinyl-carboxamido-acetamido)-benzamido)-propionsäure, Hydrochlorid, FAB 420;
aus 4-(4-CBZ-amidino-1-piperazinyl-acetyl)-1-piperazinylessigsäure-benzylester (Rf 0,44 (Dichlormethan/Methanol 9:1); erhältlich durch Kondensation von 4-CBZ-amidino-1-piperazinyl-essigsäure mit 1-Piperazinyl-essigsäure-benzylester):
   4-(4-Amidino-1-piperazinyl-acetyl)-1-piperazinyl-essigsäure, F. 272° (Zers.);
aus 3-(3-(4-CBZ-2-oxo-1-piperazinyl-acetamido)-benzamido)-propionsäure-benzylester (FAB 573; erhältlich durch Kondensation von 4-CBZ-2-oxo-1-piperazinyl-essigsäure mit 3-(3-Aminobenzamido)-propionsäure-benzylester:
   3-(3-(2-Oxo-1-piperazinyl-acetamido)-benzamido)-propionsäure, F. 225°;
aus 3-(4-CBZ-2-oxo-1-piperazinyl-acetamido)-benzamido-essigsäure-benzylester (FAB 559; erhältlich aus 3-(4-CBZ-2-oxo-1-piperazinyl-acetamido)-benzoesäure und Glycin-benzylester) :
   3-(2-Oxo-1-piperazinyl-acetamido) -benzamido-essigsäure, F. 167 °;
aus 3-(4-CBZ-amidino-2-oxo-1-piperazinyl-acetamido)-benzamido-essigsäure-benzylester (FAB 601; erhältlich durch Kondensation von 3-(4-CBZ-amidino-2-oxo-1-piperazinylacetamido)-benzoesäure mit Glycin-benzylester):
   3-(4-Amidino-2-oxo-1-piperazinyl-acetamido)-benzamido-essigsäure, F. > 300°;
aus 3-(3-(4-CBZ-2-oxo-1-piperazinyl-acetamido)-benzamido)-propionsäure-benzylester (FAB 573; erhältlich aus 3-(4-CBZ-2-oxo-1-piperazinyl-acetamido)-benzoesäure und β-Alanin-benzylester) :
   3-(3-(2-Oxo-1-piperazinyl-acetamido)-benzamido)-propionsäure, FAB 349;
aus 3-(3-(4-CBZ-amidino-2-oxo-1-piperazinyl-acetamido)-benzamido)-propionsäure-benzylester (FAB 615; erhältlich durch Kondensation von 3-(4-CBZ-amidino-2-oxo-1-piperazinylacetamido)-benzoesäure mit β-Alanin-benzylester) :
   3-(3-(4-Amidino-2-oxo-1-piperazinyl-acetamido)-benzamido)-propionsäure, F. 283° (Zers.) ;
aus 4-(4-CBZ-1-piperazinyl-carboxamido-acetyl)-1-piperazinylessigsäure-benzylester (FAB 538; erhältlich durch Kondensation von 4-CBZ-1-piperazinyl-carboxamido-essigsäure mit 1-Piperazinyl-essigsäure-benzylester):
   4-(1-Piperazinyl-carboxamido-acetyl)-1-piperazinyl-essigsäure, F. 150° (Zers.);
aus (4-(4-CBZ-amidino-1-piperazinyl-carboxamido-acetyl)-1-piperazinyl-essigsäure-benzylester (FAB 580; erhältlich durch Kondensation von 4-CBZ-amidino-1-piperazinyl-carboxamido-essigsäure mit 1-Piperazinyl-essigsäure-benzylester):
   4-(4-Amidino-1-piperazinyl-carboxamido-acetyl)-1-piperazinyl-essigsäure, F. 190-195° (Zers.);
aus 3-(4-CBZ-1-piperazinyl-carboxamido-acetamido)-benzamidoessigsäure-benzylester (FAB 588; erhältlich aus 3-(4-CBZ-1-piperazinyl-carboxamido-acetamido)-benzoesäure und Glycin-benzylester):
   3-(1-Piperazinyl-carboxamido-acetamido)-benzamido-essigsäure, F. 188°;
aus 3-(4-CBZ-amidino-1-piperazinyl-carboxamido-acetamido)benzamido-essigsäure-benzylester (FAB 630; erhältlich durch Kondensation von 4-CBZ-amidino-1-piperazinyl-carboxamido-essigsäure mit 3-Amino-benzamidoessigsäure-benzylester:
   3-(4-Amidino-1-piperazinyl-carboxamido-acetamido)-benzamido-essigsäure, F. 234°;
aus 3-(4-CBZ-amidino-1-piperazinyl-acetamido)-benzamido-essigsäure-benzylester (FAB 587; erhältlich durch Kondensation von 3-(4-CBZ-amidino-1-piperazinyl-acetamido)-benzoesäure mit Glycin-benzylester):
   3-(4-Amidino-1-piperazinyl-acetamido)-benzamido-essigsäure, F. 180-185° (Zers.);
aus 4-(4-(4-CBZ-amidino-1-piperazinyl-acetyl)-1-piperazinyl)buttersäure-benzylester (FAB 565; erhältlich durch Kondensation von 4-CBZ-amidino-1-piperazinyl-essigsäure (F. 124°) mit 4-(1-Piperazinyl)-buttersäure-benzylester):
   4-(4-(4-Amidino-1-piperazinyl-acetyl)-1-piperazinyl)-buttersäure, F. 253° (Zers.);
aus 3-(3-(4-CBZ-l-piperazinyl-carboxamido)-benzamido)-propionsäure-benzylester (FAB 545; erhältlich durch Kondensation von 3-(4-CBZ-1-piperazinyl-carboxamido)-benzoesäure und β-Alanin-benzylester) :
   3-(3-(1-Piperazinyl-carboxamido)-benzamido)-propionsäure, FAB 321;
aus 3-(3-(4-CBZ-amidino-l-piperazinyl-carboxamido)-benzamido)-propionsäure-benzylester (FAB 587; erhältlich durch Kondensation von 3-(4-CBZ-amidino-1-piperazinyl-carboxamido)benzoesäure mit β-Alanin-benzylester) :
   3-(3-(4-Amidino-1-piperazinyl-carboxamido)-benzamido)propionsäure, F. 234° (Zers.) ;
aus 3-(4-CBZ-1-piperazinyl-carboxamido)-benzamido-essigsäure-benzylester (FAB 531; erhältlich durch Kondensation von 3-(4-CBZ-1-piperazinyl-carboxamido)-benzoesäure mit Glycin-benzylester):
   3-(1-Piperazinyl-carboxamido)-benzamido-essigsäure, FAB 307;
aus 3-(4-CBZ-amidino-1-piperazinyl-carboxamido)-benzamido-essigsäure-benzylester (FAB 573; erhältlich durch Kondensation von 3-(4-CBZ-amidino-1-piperazinyl-carboxamido)-benzoesäure mit Glycin-benzylester):
   3-(4-Amidino-1-piperazinyl-carboxamido)-benzamido-essigsäure, F. 117°;
aus N-(3-(4-CBZ-2-oxo-1-piperazinyl-acetamido)-benzoyl)-L-asparaginsäure-dibenzylester (FAB 707; erhältlich durch Kondensation von 3-(4-CBZ-2-oxo-1-piperazinyl-acetamido)-benzoesäure mit L-Asparaginsäure-dibenzylester) :
   N-(3-(2-Oxo-1-piperazinyl-acetamido)-benzoyl)-L-asparaginsäure, FAB 393;
aus N-(3-(4-CBZ-amidino-2-oxo-1-piperazinyl-acetamido)-benzoyl)-L-asparaginsäure-dibenzylester (FAB 749; erhältlich durch Kondensation von 3-(4-CBZ-amidino-2-oxo-1-piperazinylacetamido)-benzoesäure mit L-Asparaginsäure-dibenzylester) :
   N-(3-(4-Amidino-2-oxo-1-piperazinyl-acetamido)-benzoyl)-L-asparaginsäure, F. 179°;
aus 4-(3-(4-CBZ-1-piperazinyl-carboxamido)-benzamido)-buttersäure-benzylester (FAB 559; erhältlich durch Kondensation von 3-(4-CBZ-1-piperazinyl-carboxamido)-benzoesäure mit 4-Aminobuttersäure-benzylester) :
   4-(3-(1-Piperazinyl-carboxamido)-benzamido)-buttersäure, FAB 335;
aus 4- (3-(4-CBZ-amidino-1-piperazinyl-carboxamido)-benzamido)-buttersäure-benzylester (FAB 601; erhältlich durch Kondensation von 3-(4-CBZ-amidino-1-piperazinyl-carboxamido)-benzoesäure mit 4-Aminobuttersäure-benzylester) :
   4-(3-(4-Amidino-1-piperazinyl-carboxamido)-benzamido)-buttersäure, F. 215°;
aus 4-(3-(4-CBZ-2-oxo-1-piperazinyl-acetamido)-benzamido)-buttersäure-benzylester (FAB 587; erhältlich durch Kondensation von 3-(4-CBZ-2-oxo-1-piperazinyl-acetamido)-benzoesäure mit 4-Aminobuttersäure-benzylester) :
   4-(3-(2-Oxo-1-piperazinyl-acetamido)-benzamido)-buttersäure, FAB 363;
aus 4-(3-(4-CBZ-amidino-2-oxo-1-piperazinyl-acetamido)-benzamido)-buttersäure-benzylester (FAB 629; erhältlich durch Kondensation von 3-(4-CBZ-amidino-2-oxo-1-piperazinylacetamido)-benzoesäure mit 4-Aminobuttersäure-benzylester) :
   4-(3-(4-Amidino-2-oxo-1-piperazinyl-acetamido) -benzamido)-buttersäure, F. 269°;
aus 4-(3-(4-CBZ-amidino-1-piperazinyl-acetamido)-benzamido)-buttersäure-benzylester (FAB 615; erhältlich durch Kondensation von 3-(4-CBZ-amidino-1-piperazinyl-acetamido)-benzoesäure mit 4-Aminobuttersäure-benzylester):
   4- (3- (4-Amidino-1-piperazinyl-acetamido) -benzamido) -buttersäure, F. 115°;
aus 3-(3-(4-CBZ-amidino-1-piperazinyl)-propionamido)-benzamido-essigsäure-benzylester (FAB 601; erhältlich durch Kondensation von 3-(3-(4-CBZ-amidino-1-piperazinyl)-propionamido)-benzoesäure mit Glycin-benzylester:
   3-(3-(4-Amidino-l-piperazinyl)-propionamido)-benzamidoessigsäure, FAB 377;F. 268°;
aus 3- (3- (3- (4-CBZ-amidino-1-piperazinyl) -propionamido) -benzamido)-propionsäure-benzylester (FAB 615; erhältlich durch Kondensation von 3-(3-(4-CBZ-amidino-1-piperazinyl)-propionamido)-benzoesäure mit β-Alanin-benzylester) :
   3-(3-(3-(4-Amidino-1-piperazinyl)-propionamido)-benzamido)-propionsäure, FAB 391; F.200° ;
aus 3- (4- (4-CBZ-amidino-1-piperazinyl-carboxamido-acetyl) -1-piperazinyl)-propionsäure-benzylester (FAB 594; erhältlich durch Kondensation von 4-CBZ-amidino-1-piperazinyl-carboxamido-essigsäure mit 3-(1-Piperazinyl)-propionsäure-benzylester) :
   3- (4- (4-Amidino-1-piperazinyl-carboxamido-acetyl) -1-piperazinyl)-propionsäure, FAB 370; F.141° ;
aus 3- (4- (4-CBZ-amidino-1-piperazinyl-acetyl) -1-piperazinyl)-propionsäure-benzylester (FAB 551; erhältlich durch Kondensation von 4-CBZ-amidino-1-piperazinyl-essigsäure mit 3-(1-Piperazinyl) -propionsäure-benzylester) :
   3-(4-(4-Amidino-1-piperazinyl-acetyl)-1-piperazinyl-propionsäure, F. 280°;
aus 3-(3-(4-CBZ-amidino-1-piperazinyl-acetamido)-benzamido)-propionsäure-methylester (FAB 525; erhältlich durch Kondensation von 4-CBZ-amidino-1-piperazinyl-essigsäure mit 3-(3-A-mino-benzamido)-propionsäure-methylester:
   3-(3-(4-Amidino-1-piperazinyl-acetamido)-benzamido)-propionsäure-methylester, Dihydrochlorid, F. 222°.

### Beispiel 2

(a) Ein Gemisch von 1,86 g 4-Amino-1-piperazinyl-essigsäure, 2,22 g 3-(3-Aminobenzamido)-propionsäure-methylester, 1,92 g DAPECI-hydrochlorid, 1,01 g N-Methylmorpholin und 70 ml DMF wird 16 Std. bei 20° gerührt. Man dampft ein, arbeitet mit Ethylacetat/5%ig. NaHCO₃-Lösung auf und erhält 3-(3-(4-Amidino-1-piperazinyl-acetamido)-benzamido)-propionsäure-methylester. Dihydrochlorid, F. 222°.
Analog erhält man durch Kondensation:
von 3-(1-Piperazinyl-carboxamido-acetamido)-benzoesäure mit Glycin-methylester:
   3-(1-Piperazinyl-carboxamido-acetamido) -benzamido-essigsäure-methylester;
aus 3-(4-Amidino-1-piperazinyl-carboxamido-acetamido)-benzoesäure und β-Alanin-methylester:
   3-(3- (4-Amidino-1-piperazinyl-carboxamido-acetamido)-benzamido)-propionsäure-methylester;
von 4-Amidino-1-piperazino-essigsäure mit 1-Piperazinyl-essigsäure-methylester:
   4- (4-Amidino-1-piperazinyl-acetyl)-1-piperazinyl-essigsäure-methylester;
von 3-(2-Oxo-1-piperazinyl-acetamido)-benzoesäure mit β-Alanin-methylester:
   3-(3-(2-Oxo-1-piperazinyl-acetamido)-benzamido)-propionsäure-methylester;
von 3-(4-Amidino-2-oxo-1-piperazinyl-acetamido)-benzoesäure mit Glycin-methylester:
   3-(4-Amidino-2-oxo-1-piperazinyl-acetamido)-benzamido-essigsäure-methylester;
von 3-(4-Amidino-2-oxo-1-piperazinyl-acetamido)-benzoesäure mit β-Alanin-methylester:
   3-(3-(4-Amidino-2-oxo-1-piperazinyl-acetamido)-benzamido)-propionsäure-methylester;
von 4-Amidino-1-piperazinyl-carboxamido-essigsäure mit 1-Piperazinyl-essigsäure-methylester:
   4-(4-Amidino-1-piperazinyl-carboxamido-acetyl) -1-piperazinyl-essigsäure-methylester;
von 3-(4-Amidino-1-piperazinyl-carboxamido-acetamido)-benzoesäure mit Glycin-methylester:
   3-(4-Amidino-1-piperazinyl-carboxamido-acetamido)-benzamido-essigsäure-methylester;
von 3-(4-Amidino-1-piperazinyl-acetamido)-benzoesäure mit Glycin-methylester:
   3-(4-Amidino-1-piperazinyl-acetamido)-benzamido-essigsäure-methylester;
von 4-Amidino-1-piperazinyl-essigsäure mit 4-(1-Piperazinyl)-buttersäure-methylester:
   4-(4-(4-Amidino-1-piperazinyl-acetyl)-1-piperazinyl)-buttersäure-methylester;
von 3-(4-Amidino-1-piperazinyl-carboxamido)-benzoesäure mit β-Alanin-methylester:
   3-(3-(4-Amidino-1-piperazinyl-carboxamido)-benzamido)-propionsäure-methylester;
von 3-(4-Amidino-1-piperazinyl-carboxamido)-benzoesäure mit Glycin-methylester:
   3-(4-Amidino-1-piperazinyl-carboxamido)-benzamido-essigsäure-methylester;
von 3-(4-Amidino-2-oxo-1-piperazinyl-acetamido)-benzoesäure mit L-Asparaginsäure-dimethylester:
   N- (3- (4-Amidino-2-oxo-1-piperazinyl-acetamido) -benzoyl)-L-asparaginsäure-dimethylester;
von 3-(4-Amidino-1-piperazinyl-carboxamido)-benzoesäure mit 4-Aminobuttersäure-methylester:
   4-(3-(4-Amidino-1-piperazinyl-carboxamido)-benzamido)-buttersäure-methylester;
von 3-(4-Amidino-2-oxo-1-piperazinyl-acetamido)-benzoesäure mit 4-Aminobuttersäure-methylester:
   4-(3-(4-Amidino-2-oxo-1-piperazinyl-acetamido)-benzamido)-buttersäure-methylester;
von 3-(4-Amidino-2-oxo-1-piperazinyl-acetamido)-benzoesäure mit 4-Aminobuttersäure-ethylester:
   4-(3-(4-Amidino-2-oxo-1-piperazinyl-acetamido)-benzamido-buttersäure-ethylester;
von 3-(3-(4-Amidino-1-piperazinyl)-propionamido)-benzoesäure mit Glycin-methylester:
   3-(3-(4-Amidino-1-piperazinyl)-propionamido)-benzamidoessigsäure-methylester;
von 3-(3-(4-Amidino-1-piperazinyl)-propionamido)-benzoesäure mit β-Alanin-methylester:
   3-(3-(3-(4-Amidino-1-piperazinyl)-propionamido)-benzamido)-propionsäure-methylester;
von 4-Amidino-1-piperazinyl-carboxamido-essigsäure mit 3-(1-Piperazinyl)-propionsäure-methylester:
   3-(4-(4-Amidino-1-piperazinyl-carboxamido-acetyl)-1-piperazinyl)-propionsäure-methylester;
von 4-Amidino-1-piperazinyl-essigsäure mit 3-(1-Piperazinyl)-propionsäure-methylester:
   3-(4-(4-Amidino-1-piperazinyl-acetyl)-1-piperazinyl)-propionsäure-methylester;
von 3-(4-Amidino-1-piperazinyl-acetyl)-benzoesäure mit β-Alanin-ethylester:
   3-(3-(4-Amidino-1-piperazinyl-acetamido)-benzamido)-propionsäure-ethylester.

### Beispiel 3

Eine Lösung von 201 mg 1-Amidino-3,5-dimethylpyrazol-nitrat in 17 ml Dioxan und 5 ml Wasser wird mit 0,17 ml Ethyl-diisopropylamin versetzt und 15 Min. gerührt. Man gibt dann 357 mg 3-(3-(1-Piperazinyl-carboxamido)-benzamido)-propionsäurehydrochlorid (FAB 321; erhältlich durch Reaktion von 3-(3-Aminobenzamido)-propionsäure-benzylester mit Diphosgen zu 3-(3-Isocyanato-benzamido)-propionsäure-benzylester, Anlagerung von 1-BOC-piperazin zu 3-(3-(4-BOC-1-piperazinyl-carboxamido)-benzamido)-propionsäure-benzylester, Abspaltung der BOC-Gruppe mit 4 n HCl in Dioxan zu 3-(3-(1-Piperazinyl-carboxamido)-benzamido)-propionsäure-benzylester-hydrochlorid (FAB 411) und Hydrogenolyse an 5%ig. Pd-C) und weitere 0,17 ml Ethyl-diisopropylamin hinzu, kocht das Gemisch 45 Stunden, dampft ein, löst den Rückstand in Wasser, wäscht die Lösung mit Ether, dann mit Ethylacetat, dampft erneut ein und erhält 3-(3-(4-Amidino-1-piperazinyl-carboxamido)-benzamido)-propionsäure, F. 234° (Zers.).

Analog erhält man aus den entsprechenden Piperazinderivaten die in Beispiel **1.** angegebenen 4-Amidino-piperazinderivate.

### Beispiel 4

Analog Beispiel 3 erhält man mit 1-Amidino-3,5-dimethyl-pyrazolnitrat:
aus 3-(4-(1-Piperazinyl-carboxamido-acetyl)-1-piperazinyl)-propionsäure:
   3-(4-(4-Amidino-1-piperazinyl-carboxamido-acetyl)-1-piperazinyl)-propionsäure, F. 141°;
aus 3-(3-(1-Piperazinyl)-propionamido)-benzamido-essigsäure:
   3-(3-(4-Amidino-1-piperazinyl)-propionamido)-benzamido-essigsäure, F. 268°;
aus 3-(3-(3-(1-Piperazinyl)-propionamido)-benzamido)-propionsäure:
   3-(3-(3-(4-Amidino-1-piperazinyl)-propionamido)-benzamido)-propionsäure, F. 200°;
aus 3-(3-(2-(1-Piperazinyl)-propionamido)-benzamido)-propionsäure:
   3-(3-(2-(4-Amidino-1-piperazinyl)-propionamido)-benzamido)-propionsäure, F. 171°;
aus 3-(3-(1-Piperazinyl-acetamido)-benzamido)-propionsäure:
   3-(3-(4-Amidino-1-piperazinyl-acetamido)-benzamido)-propionsäure, Dihydrochlorid, F. 272°.

Die nachfolgenden Beispiele betreffen pharmazeutische Zubereitungen.

### Beispiel A: Tabletten

Ein Gemisch von 1 kg Wirkstoff der Formel I, 4 kg Lactose, 1,2 kg Maisstärke, 200 g Talk und 100 g Magnesiumstearat wird in üblicher Weise zu Tabletten verpreßt, derart, daß jede Tablette 100 mg Wirkstoff enthält.

### Beispiel B: Dragees

Analog Beispiel A werden Tabletten gepreßt, die anschließend in üblicher Weise mit einem Überzug aus Saccharose, Maisstärke, Talk, Tragant und Farbstoff überzogen werden.

### Beispiel C: Kapseln

500 g Wirkstoff der Formel I werden in üblicher Weise in Hartgelatinekapseln gefüllt, so daß jede Kapsel 500 mg Wirkstoff enthält.

### Beispiel D: Injektionsgläser

Eine Lösung von 100 g Wirkstoff der Formel I in 4 l zweifach destilliertem Wasser wird mit 2 n Salzsäure auf pH 6,5 eingestellt, steril filtriert und in Injektionsgläser abgefüllt. Man lyophilisiert unter sterilen Bedingungen und verschließt steril. Jedes Injektionsglas enthält 50 mg Wirkstoff.

### Beispiel E: Suppositorien

Man schmilzt ein Gemisch von 50 g Wirkstoff der Formel I mit 10 g Sojalecithin und 140 g Kakaobutter, gießt in Formen und läßt erkalten. Jedes Suppositorium enthält 250 mg Wirkstoff.

## Patentansprüche

1. Verbindung der Formel I
Y-(CₘH₂ₘ-CHR¹)ₙ-CO-(NH-CHR²-CO)ᵣ-Z I
worin
Y 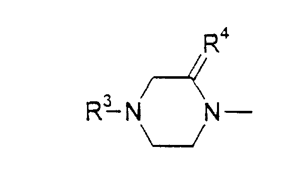
Z 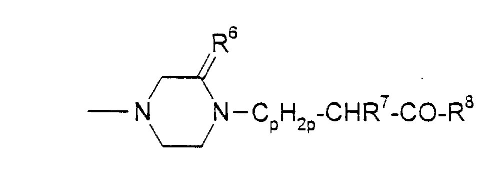 oder 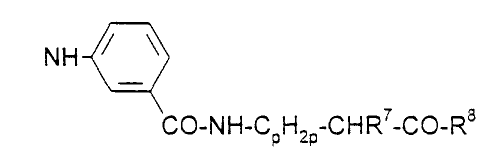
R¹, R² und R⁷ jeweils -CₜH₂ₜ-R⁹, Benzyl, Hydroxybenzyl, Imidazolylmethyl oder Indolyl-methyl,
R³ H oder H₂N-C(=NH)-,
R⁴ und R⁶ jeweils (H, H) oder =O,
R⁸ OH, OA oder NHOH,
R⁹ H, OH, NH₂, SH, SA, COOH, CONH₂ oder NH-C(=NH)-NH₂,
A jeweils Alkyl mit 1-4 C-Atomen,
m und t jeweils 0, 1, 2, 3 oder 4
n und r jeweils 0 oder 1, und
P 0, 1 oder 2 bedeuten,
worin ferner die Piperazinringe duch 1 bis 4 Gruppen A substituiert sein können,
sowie deren Salze.

2. 3-(3-(4-Amidino-1-piperazinyl-acetamido)-benzamido)-propionsäure gemäß Anspruch 1.

3. Verfahren zur Herstellung einer Verbindung der Formel I nach Patentanspruch 1 sowie von ihren Salzen, dadurch gekennzeichnet, daß man
(a) eine Verbindung der Formel I aus einem ihrer funktionellen Derivate durch Behandeln mit einem solvolysierenden oder hydrogenolysierenden Mittel in Freiheit setzt, oder daß man
(b) eine Carbonsäure der Formel II
Y-(CₘH₂ₘ-CHR¹)ₙ-CO-G¹-OH II
worin
G¹
(a) fehlt,
(b) -NH-CHR²-CO- bedeutet,
(c) -(NH-CHR²-CO)ᵣ 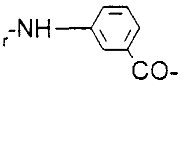 bedeutet,
Y, R¹, R², m, n und r die oben angegebenen Bedeutungen haben,
oder eines ihrer reaktionsfähigen Derivate
mit einer Aminoverbindung der Formel III
H-G² III
worin
G²
(a) -(NH-CH²-CO)ᵣ-Z bedeutet,
(b) Z bedeutet,
(c) -NH-CₚH₂ₚ-CHR⁷-CO-R⁸ bedeutet, und
Z, R², R⁷, R⁸, r und p die oben angegebenen Bedeutungen haben,
umsetzt,
oder daß man
(c) zur Herstellung einer Verbindung der Formel I, worin R³ H₂N-C(=NH)- bedeutet, an ein Nitril, das der Formel I entspricht, aber an Stelle des Restes R³ eine CN-Gruppe enthält, Ammoniak anlagert,
und/oder daß man gegebenenfalls eine Carbonsäure der Formel I (R⁸ = OH) in einen entsprechenden Ester (I, R⁸ = OA) oder in die entsprechende Hydroxamsäure (I, R⁸ = NHOH) überführt und/oder ein H-Atom durch Behandeln mit einem amidinierenden Mittel durch eine Amidinogruppe ersetzt oder einen Ester der Formel I (R⁸ = OA) verseift und/oder eine Verbindung der Formel I durch Behandeln mit einer Säure oder einer Base in eines ihrer Salze überführt.

4. Verfahren zur Herstellung pharmazeutischer Zubereitungen, dadurch gekennzeichnet, daß man eine Verbindung der Formel I nach Anspruch 1 und/oder eines ihrer physiologisch unbedenklichen Salze zusammen mit mindestens einem festen, flüssigen oder halbflüssigen Träger- oder Hilfsstoff in eine geeignete Dosierungsform bringt.

5. Pharmazeutische Zubereitung, gekennzeichnet durch einen Gehalt an mindestens einer Verbindung der Formel I nach Anspruch 1 und/oder einem ihrer physiologisch unbedenklichen Salze.

6. Verwendungvon Verbindungen der Formel I nach Anspruch 1 oder von deren physiologisch unbedenklichen Salzen zur Herstellung eines Arzneimittels.

## Claims

1. A compound of formula I
Y- (CₘH₂ₘ-CHR¹)ₙ-CO-(NH-CHR²-CO)ᵣ-Z I
Wherein
Y is 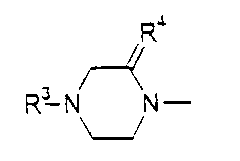
Z is 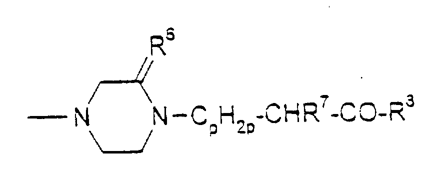
or 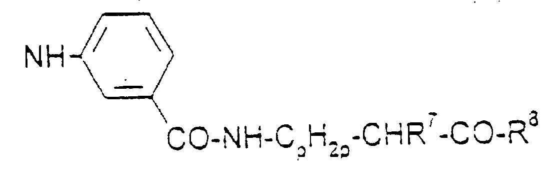
R¹, R² and R⁷ are each -CₜH₂ₜ-R⁹, benzyl, hydroxybenzyl, imidazolylmethyl or indolylmethyl,
R³ is H or H₂N-C(=NH)-,
R⁴ and R⁶ are each (H,H) or =O,
R⁸ is OH, OA or NHOH,
R⁹ is H, OH, NH₂, SH, SA, COOH, CONH₂ or NH-C(=NH)-NH₂,
A is in each case alkyl having 1-4 C atoms,
m and t are each 0, 1, 2, 3 or 4,
n and r are each 0 or 1 and
P is 0, 1 or 2,
and wherein the piperazine rings can be substituted by 1 to 4 groups A,
and its salts.

2. 3-(3-(4-Amidinopiperazin-1-ylacetamido)benzamido)-propionic acid according to Claim 1.

3. A process for the preparation of a compound of formula I according to Claim 1, and its salts, characterised in that
(a) a compound of formula I is freed from one of its functional derivatives by treatment with a solvolysing or hydrogenolysing agent, or
(b) a carboxylic acid of formula II
Y-(CₘH₂ₘ-CHR¹)ₙ-CO-G¹-OH II
wherein
G¹
(a) is absent,
(b) is -NH-CHR²-CO- or
(c) is 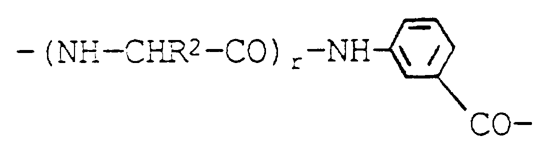
Y, R¹, R², m, n and r are defined as indicated above,
or one of its reactive derivatives,
is reacted with an amino compound of formula III:
H-G² III
wherein
G²
(a) is -(NH-CHR²-CO)ᵣ-Z,
(b) is Z or
(c) is -NH-CₚH₂ₚ-CHR⁷-CO-R⁸ and
Z, R², R⁷, R⁸, r and p are defined as indicated above,
or
(c) to prepare a compound of formula I wherein R³ is H₂N-C(=NH)-, ammonia is added on to a nitrile which has formula I except that it contains a CN group in place of the radical R³,
and/or, if appropriate, a carboxylic acid of formula I (R⁸ = OH) is converted to a corresponding ester (I, R⁸ = OA) or to the corresponding hydroxamic acid (I, R⁸ = NHOH), and/or an H atom is replaced with an amidino group by treatment with an amidine-forming agent, or an ester of formula I (R⁸ = OA) is saponified, and/or a compound of formula I is converted to one of its salts by treatment with an acid or a base.

4. A process for the manufacture of pharmaceutical preparations, characterised in that a compound of formula I according to Claim 1, and/or one of its physiologically acceptable salts, are converted to a suitable dosage form together with at least one solid, liquid or semiliquid excipient or adjunct.

5. A pharmaceutical preparation, characterised in that it contains at least one compound of formula I according to Claim 1, and/or one of its physiologically acceptable salts.

6. Use of compounds of formula I according to Claim 1, or their physiologically acceptable salts, for the preparation of a drug.

## Revendications

1. Composé de formule I :
Y-(CₘH₂ₘ-CHR¹)ₙ-CO-(NH-CHR²-CO)ᵣ-Z (I)
où
Y représente 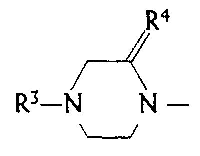
Z représente 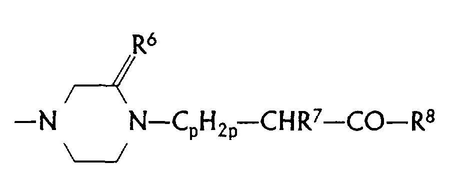 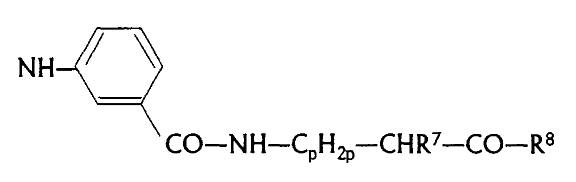
R¹, R² et R⁷ représentent -CₜH₂ₜ-R⁹, le benzyle, l'hydroxybenzyle, l'imidazolylméthyle ou l'indolylméthyle,
R³ représente H ou H₂N-C(=NH)-,
R⁴ et R⁶ représentent (H,H) ou =O,
R⁸ représente OH, OA ou NHOH,
R⁹ H, OH, NH₂, SH, SA, COOH, CONH₂ ou NH-C(=NH)-NH₂,
A un alkyle comportant 1 à 4 atomes de C,
m et t sont égaux à 0, 1, 2, 3 ou 4,
n et r sont égaux à 0 ou 1 et
p est égal à 0, 1 ou 2,
les cycles pipéraziniques pouvant être, en outre, substitués par 1 à 4 groupes A, ainsi que leurs sels.

2. L'acide 3-(3-(4-amidino-1-pipérazinylacétamido)-benzamido)-propionique selon la revendication 1.

3. Procédé pour la préparation d'un composé de formule I selon la revendication 1, ainsi que de leurs sels, caractérisé
(a) en ce que l'on libère un composé de formule I de l'un de ses dérivés fonctionnels par traitement avec un agent solvolysant ou hydrogénolysant ou
(b) en ce que l'on fait réagir un acide carboxylique de formule II :
Y-(CₘH₂ₘ-CHR¹)ₙ-CO-G¹-OH (II)
où
G¹
(a) manque,
(b) représente-NH-CHR²-CO-
(c) représente -(NH-CHR²-CO)ᵣ 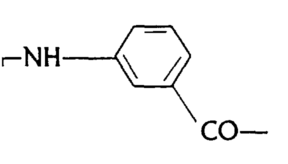
Y, R¹, R², m, n et r ayant les significations indiquées ci-dessus,
ou l'un de ses dérivés réactifs,
avec un composé aminé de formule III :
H-G² (III)
où
G²
(a) représente -(NH-CH²-CO)ᵣ-Z,
(b) représente Z,
(c) représente -NH-CₚH₂ₚ-CHR⁷-CO-R⁸ et
Z, R², R⁷, R⁸, r et p ayant les significations indiquées ci-dessus,
ou
(c) en ce que, pour la préparation d'un composé de formule I où R³ représente H₂N-C(=NH)-, l'on fixe par addition sur un nitrile correspondant à la formule I, mais contenant à la place du reste R³ un groupe CN, de l'ammoniac,
et/ou en ce que l'on transforme éventuellement un acide carboxylique de formule I (R⁸=OH) en un ester correspondant (I, R⁸=OA) ou en l'acide hydroxamique correspondant (I, R⁸=NHOH) et/ou en ce que l'on remplace un atome de H par un groupe amidino par traitement avec un agent amidinant ou en ce que l'on saponifie un ester de formule I (R⁸=OA) et/ou en ce que l'on transforme un composé de formule I en l'un de ses sels par traitement avec un acide ou une base.

4. Procédé pour la fabrication de préparations pharmaceutiques, caractérisé en ce que l'on met sous une forme de dosage appropriée un composé de formule I selon la revendication 1 et/ou l'un de ses sels physiologiquement acceptables, associé à au moins un support ou un adjuvant solide, liquide ou semi-liquide.

5. Préparation pharmaceutique caractérisée en ce qu'elle contient au moins un composé de formule I selon la revendication 1 et/ou l'un de ses sels physiologiquement acceptables.

6. Utilisation des composés de formule I selon la revendication 1 ou de leurs sels physiologiquement acceptables pour la fabrication d'un médicament.
